(19) Europäisches Patentamt
European Patent Office
Office européen des brevets

(11) Publication number: 0 283 151 A2

# (12) EUROPEAN PATENT APPLICATION

(21) Application number: 88301645.3

(51) Int. Cl.[4]: A61K 31/557

(22) Date of filing: 25.02.88

Claims for the following Contracting States: ES + GR.

(30) Priority: 27.02.87 US 19833

(43) Date of publication of application: 21.09.88 Bulletin 88/38

(84) Designated Contracting States: AT BE CH DE ES FR GB GR IT LI LU NL SE

(71) Applicant: ALLERGAN INC
2525 Dupont Drive
Irvine California 92715(US)

(72) Inventor: Woodward, David F.
23152 Tulip
El Toro California 92630(US)

(74) Representative: Waters, David Martin, Dr. et al
Smith Kline & French Laboratories Ltd.
Patent Department Mundells
Welwyn Garden City Hertfordshire AL7 1EY(GB)

## (54) Prostaglandins useful for lowering intraocular pressure.

(57) Compounds of the formula

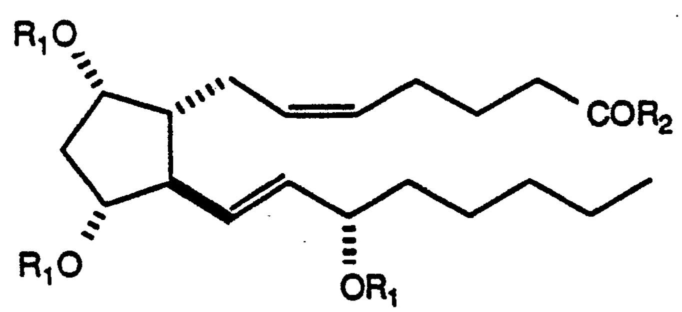

where $R_1$ is acyl of 1-7 carbon atoms and $R_2$ is -OH, -$OR_3$ where $R_3$ is alkyl of 1 to 6 carbon atoms, or $-O^-X^+$ where $X^+$ is a pharmaceutically acceptable cation, are highly active ocular hypotensive agents.

EP 0 283 151 A2

# Prostaglandins Useful for Lowering Intraocular Pressure

## Background

This invention relates to $PGF_{2\alpha}$ esters which are potent ocular hypotensive agents. More specifically, this invention relates to the 9,11,15-triesters of $PGF_{2\alpha}$.

## Related Art

The parent compound $PGF_{2\alpha}$ is a known compound. It has been reported that $PGF_{2\alpha}$ and its C-1 carboxylic acid esters affect intraocular pressure when applied topically to the eye (Bito, L. Z., Exp. Eye Res. (1984) 38, 181). The 9,11,15-triacetate of $PGF_{2\alpha}$ has been reported in several publications, eg. Lukacs, G, et al, Tetrahedron Letters, 7, 515, 1973.

## Summary of the Invention

This invention relates to a method for lowering intraocular pressure which method comprises administering to an eye a composition having a therapeutically effective amount of a compound of the formula

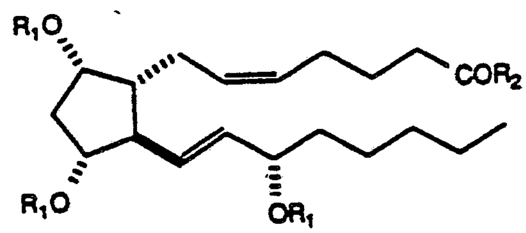

where $R_1$ is an acyl radical of 1 to 7 carbon atoms and $R_2$ is -OH, $-OR_3$ where $R_3$ is lower alkyl of 1 to 6 carbon atoms or $-O^-X^+$ where $X^+$ is a pharmaceutically acceptable cation.

Further, this invention relates to the use of compounds of formula I for treating ocular hypertension, more particularly for treating narrow-angle glaucoma, open-angle glaucoma, post surgical and post-laser trabeculectomy, ocular hypertensive episodes, and as a pre-surgical adjunct. In a similar vein, this invention also covers pharmaceutical formulations comprising one or more compounds of formula 1 in admixture with a pharmaceutically acceptable excipient for use in lowering intraocular pressure.

## General Embodiments

### Definitions

The term "acyl" is here used to designate the radical -COR where R is an aliphatic radical of 1 to 6 carbon atoms. This R group may be straight or branched and may be saturated or unsaturated, though an unbranched alkyl radical is preferred. "Acetyl" as used here takes its common meaning of being derived from acetic acid and having the form $-COCH_3$.

A "pharmaceutically acceptable cation" is any cation which does not modify the activity of the parent compound and does not impart any deleterious or untoward effect on the subject to which it is administered in the context in which it is used. Such a salt may be derived from an inorganic or organic base. It may be a mono or polyvalent ion. Of particular interest are the inorganic ions, sodium, potassium, calcium and magnesium. Also, ammonium salts, particularly salts prepared from mono-, di-and trialkyl amines or ethanol amines are preferred. Salts may also be formed with caffeine, tromethamine and similar molecules.

The preferred triesters of this invention are those where $R_1$ is 2-4 carbon atoms and, more particularly, the triacetyl compound (5Z,9α,13E,15S)-9,11,15-triacetoxyprosta-5,13-dienoic acid.

These triesters of $PGF_{2\alpha}$ represent potent ocular hypotensives that are valuable in treating narrow-angle glaucoma, open-angle glaucoma, post-surgical and post-laser trabeculectomy ocular hypertensive episodes, and as a pre-surgical adjunct. It has been found that acylating the three $PGF_{2\alpha}$ hydroxyl groups has a very substantial effect in decreasing intraocular pressure (IOP). About a 10-fold increase in potency in terms of lowering IOP was observed with the triacetyl compound compared to the unacylated parent, $PGF_{2\alpha}$. Stated another way, the triacetyl ester at a concentration of 0.01% has the same effect in lowering intraocular pressure as a 0.1% solution of $PGF_{2\alpha}$. In addition, the triacyl esters demonstrate substantially extended IOP lowering activity in comparison to $PGF_{2\alpha}$.

The ocular hypotensive activity of these compounds was measured by applanation pneumatonometry.

The compounds of this invention are best administered directly to the ocular cavity rather than by some systemic method of administration. Any common ocular formulation, such as a solution, suspension, gel, ointment, or extended release insert, may be used as a drug delivery means. Preparation of such formulations are well described in the art as exemplified, for example, by <u>Remington's Pharmaceutical Sciences</u>, Edition 17, Mack Publishing Company, Easton, Pennsylvania.

Such formulations are usually sterile and, in order to maintain sterility, can contain one or more preservatives such as benzalkonium chloride, chlorobutanol, phenylmercuric nitrate or acetate, thimerosal, methyl or propylparabens, or phenylethanol. Tonicity adjusting agents can also be added. Such agents typically will be employed to provide a tonicity equivalent to a 0.5-2.0% NaCl solution, preferably a 0.7-1.5% NaCl solution. Tonicity adjusting agents include NaCl, KCl, buffer salts, dextrose, glycerol and propylene glycol. The formulations can contain buffering agents such that the pH of the formulation preferably approximates to 7.4.

Solutions can be administered as eyedrops and are usually presented as a drop-dispenser containing the solution. The drop-dispenser can be a container with an accompanying bulb dropper or, more usually, it can be a dropper container formed from, for example, low density polyethylene or polypropylene and having a dispensing tip.

In order to increase the contact time of the solution with the eye, the solution can contain a viscosity enhancer such as methylcellulose or hydroxypropylmethylcellulose. The viscosity of the solutions will, in general, be in the range 15-25 centipoises.

The formulations can be presented as powders for reconstitution into solutions. Such formulations typically comprise, in addition to the powder, a sterile diluent and a sterile dropper assembly. The powder can contain a sterile inert bulking agent such as mannitol.

Ophthalmic ointment formulations will generally be presented in a collapsible tube having a dispensing tip, The collapsible tube typically is formed from an ophthalmically acceptable soft metal such as tin.

The ointment base can be formed from petrolatum and mineral oil although other substances such as polyethylene glycols, glycerol and anhydrous lanolin can also be used.

Extended release inserts include wafer-type inserts which are placed below the eyelid and release the active ingredient by slow diffusion. Diffusion-based inserts include those such as Ocusert (Trade Mark) manufactured by the Alza Corporation.

In treating open-angle or narrow-angle glaucoma, or any other of the several utilities described above, a therapeutic (effective) concentration is a concentration which reduces intraocular pressure in a mammal having elevated pressure to a level which is considered a normal and acceptable physiological range. A given therapeutic concentration will vary from condition to condition and may vary with the severity of the condition being treated and the patients' susceptibility to treatment. Accordingly, no single therapeutic concentration can be precisely pinpointed for each condition being treated but will be best determined at the time and place through routine experimentation. However, it is anticipated that in the treatment of open-angle glaucoma, an ocular formulation containing between 0.001 and 0.5 percent by weight will effectively reduce intraocular pressure to a physiologically acceptable level. Administration may be once or several times daily depending on the concentration of the solution being administered and the inherent activity of the triester being administered.

<u>Specific Embodiments</u>

These compounds are most readily made from the parent compound by any of the numerous methods known for forming esters. More specifically. the parent compound $PGF_{2\alpha}$can be treated with an appropriate acid chloride or anhydride in a solvent such as pyridine. The method described by T. W. Green in "Protective Groups In Organic Synthesis"; John Wiley and Sons, New York, 1981, pp. 53-55, serves as a reference for making these compounds.

Example 1

(5Z,9α,13E,15S)-9,11,15-triacetoxyprosta-5,13-dienoic acid

A dry, 25ml, round bottomed flask, equipped with a magnetic stir bar and reflux condenser was connected to a mineral oil bubbler to exclude moisture and air. This flask was charged with 2mL of pyridine (dried over 4 A molecular sieves), 2 mL of acetic anhydride and $PGF_{2\alpha}$(54.5 mg, 0.154 mmol). The reaction mixture was heated to 50°C for 2 hours. After 2 hours at 50°C, the reaction mixture was concentrated in vacuo. The residue (light yellow oil) was dissolved in ethyl acetate (5 ml) and washed with 1N HCl (2 mL) to pH 3. The ethyl acetate layer was washed with $H_2O$ (2 mL) and saturated aqueous NaCl (2 mL) before drying over $MgSO_4$ and being concentrated in vacuo. The colorless oil was chromatographed (HPLC: Whatman M-9, ODS-2, 10 mm × 50 cm column; 70:30 $CH_3CN$:$H_2O$ elution, 4 mL/min, refractive index detection) to yield the title compound as a colorless oil. Mass spectral analysis of the trimethylsilyl derivative ($C_{29}H_{48}O_8Si$) confirmed the presence of the title structure: 552 (m/e), 537, 492, 432, 372.

Proceeding in a similar manner, but substituting the appropriate anhydride or acid chloride, the following compounds, for example, can be prepared:

(5Z,9α,13E,15S)-9,11,15-tripropionyloxyprosta-5,13-dienoic acid; and

(5Z,9α,13E,15S)-9,11,15-tributyryloxyprosta-5,13-dienoic acid.

Example 2

IOP Effects of $PGF_{2\alpha}$ and its Triacetyl Ester in New Zealand-Dutch Belt Cross Rabbits

The effect of 0.1 percent $PGF_{2\alpha}$ on IOP versus 0.01% solution of the 9,11,15 triacetyl ester of $PGF_{2\alpha}$was measured in New Zealand-Dutch Belt cross rabbits. Intraocular pressure was measured by applanation pneumatonometry in conscious animals. Nine animals were used for each experiment. The data from this study as summarized in Table I show about a 10-fold difference in the activity of 9,11,15-triacetyl $PGF_{2\alpha}$ compound over $PGF_{2\alpha}$.

Table I

COMPARISON OF THE EFFECT OF $PGF_{2\alpha}$ AND THE 9,11,15-TRIACETYLESTER ON INTRAOCULAR PRESSURE (RABBITS)

| | 0.01% Triester | | | | | | 0.1% PGF2α | |
|---|---|---|---|---|---|---|---|---|
| | Test | | Control | | Test | | Control | |
| Hours | IOP | (SEM) | IOP | (SEM) | IOP | (SEM) | IOP | (SEM) |
| 0 | 24.39 | (0.92) | 23.56 | (0.86) | 21.67 | (0.88) | 21.17 | (0.91) |
| 0.5 | 24.67 | (0.40) | 24.78 | (0.46) | 23.00 | (0.62) | 23.17 | (0.54) |
| 1.0 | 23.56 | (2.14) | 23.06 | (0.78) | 24.00 | (0.51) | 22.50 | (0.68) |
| 1.5 | 22.72* | (0.66) | 23.39 | (0.49) | 24.35 | (0.56) | 22.83 | (0.98) |
| 2.0 | 21.33* | (0.91) | 24.44 | (0.6) | 22.67 | (0.77) | 23.42 | (0.55) |
| 2.5 | 20.83* | (0.88) | 25.33 | (0.55) | 20.92 | (0.80) | 24.00 | (0.65) |
| 3.5 | 22.22* | (0.66) | 25.44 | (0.49) | 19.50* | (0.57) | 24.00 | (0.47) |
| 4.5 | 23.28 | (0.79) | 26.28 | (0.97) | 20.75 | (0.80) | 24.58 | (1.09) |

* $p < 0.05$

Example 4

The duration of action of the triester (9,11,15-triacetyl ester) in comparison with that of the native $PGF_{2\alpha}$ was tested in cats. To ensure that the $PGF_{2\alpha}$ would demonstrate some measurable level of action, the test was run at a concentration of 0.1%, that being used for both compounds. As demonstrated by Table II, the triacetyl ester showed a significant difference in IOP between the treated and untreated eye at between 1 and 24 hours, when the last reading was taken, while the parent $PGF_{2\alpha}$ showed a significant difference only between 1 and 4 hours.

Table II

THE EFFECT OF TOPICAL 0.1% $PGF_{2\alpha}$ (25µL) ON IOP IN CATS (N=5)

| Hours | Test IOP | Control IOP |
|---|---|---|
| -0.5 | 19.7 | 20.0 |
| 0.0 | 19.8 | 19.8 |
| 0.5 | 18.2 | 21.3 |
| 1.0 | 15.4* | 20.5 |
| 2.0 | 14.5* | 19.8 |
| 3.0 | 15.3* | 20.1 |
| 4.0 | 16.7* | 18.8 |
| 6.0 | 21.5 | 20.9 |
| 24.0 | 19.8 | 20.2 |

THE EFFECT OF TOPICAL TRIACETYL $PGF_{2\alpha}$ (0.1%, 25µL) ON IOP IN CATS

| Hours | Test IOP | Control IOP |
|---|---|---|
| -0.5 | 21.2 | 21.5 |
| 0.0 | 20.6 | 21.0 |
| 0.5 | 18.6 | 20.2 |
| 1.0 | 17.9* | 21.1 |
| 2.0 | 17.0* | 21.2 |
| 3.0 | 18.0* | 22.6 |
| 4.0 | 17.8* | 21.2 |
| 6.0 | 18.8* | 24.2 |
| 24.0 | 20.0* | 22.5 |

* $p < 0.05$

**Claims**

1. The use of a compound of the formula:

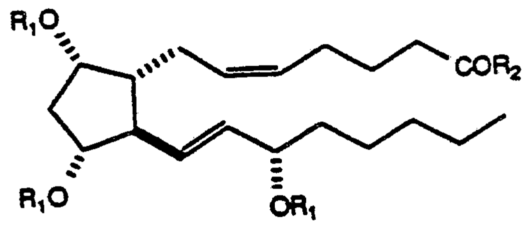

where $R_1$ is an acyl radical of 1 to 7 carbon atoms and $R_2$ is -OH, -$OR_3$ where $R_3$ is lower alkyl of 1 to 6 carbon atoms or -$O^-X^+$ where $X^+$ is a pharmaceutically acceptable cation, in the preparation of an ophthalmic medicament for lowering intraocular pressure.

2. The use according to claim 1 wherein $R_1$ has 2-4 carbon atoms.

3. The use according to claim 2 wherein $R_1$ is acetyl and $R_2$ is OH, the compound being (5Z,9α,13E,15S)-9,11,15-triacetoxyprosta-5,13-dienoic acid.

4. An ophthalmic composition adapted for only topical, to the exclusion of oral and injectable administration, comprising a compound of the formula:

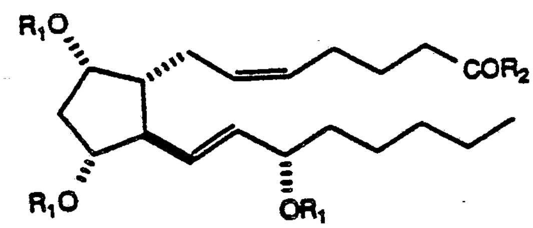

where $R_1$ is an acyl radical of 1 to 7 carbon atoms and $R_2$ is -OH, -$OR_3$ where $R_3$ is lower alkyl of 1 to 6 carbon atoms or -$O^-X^+$ where $X^+$ is a pharmaceutically acceptable cation, and an ophthalmologically acceptable carrier therefor.

5. An ophthalmic composition according to claim 4 which comprises a container for dispensing eyedrops and, contained therein, an ophthalmically acceptable solution of a compound as defined in claim 5.

6. An ophthalmic composition according to claim 4 which is an ointment.

7. A composition according to any one of claims 4 to 6 containing a compound wherein $R_1$ has 2-4 carbon atoms.

8. A composition according to claim 7 wherein $R_1$ is acetyl and $R_2$ is OH, the compound being (5Z,9α,13E,15S)-9,11,15-triacetoxyprosta-5,13-dienoic acid.

Claims for the following Contracting States GR, ES:

1. The use of a compound of the formula:

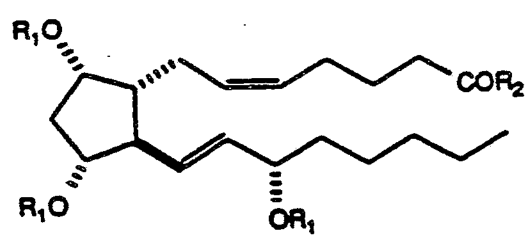

where $R_1$ is an acyl radical of 1 to 7 carbon atoms and $R_2$ is -OH, -$OR_3$ where $R_3$ is lower alkyl of 1 to 6

carbon atoms or $-O^{-}X^{+}$ where $X^{+}$ is a pharmaceutically acceptable cation, in the preparation of an ophthalmic medicament for lowering intraocular pressure.

2. The use according to claim 1 wherein $R_1$ has 2-4 carbon atoms.

3. The use according to claim 2 wherein $R_1$ is acetyl and $R_2$ is OH, the compound being (5Z,9α,13E,15S)-9,11,15-triacetoxyprosta-5,13-dienoic acid.

4. A process for preparing an ophthalmic composition adapted for only topical, to the exclusion of oral and injectable administration, comprising a compound of the formula:

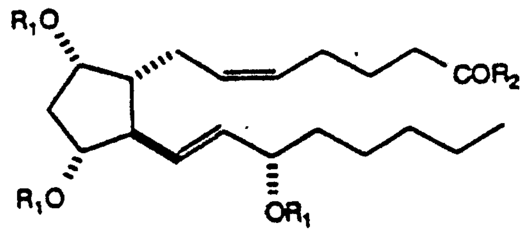

where $R_1$ is an acyl radical of 1 to 7 carbon atoms and $R_2$ is -OH, $-OR_3$ where $R_3$ is lower alkyl of 1 to 6 carbon atoms or $-O^{-}X^{+}$ where $X^{+}$ is a pharmaceutically acceptable cation, and an ophthalmologically acceptable carrier therefor, which process comprises bringing the compound into association with the ophthalmologically acceptable carrier.

5. A process for preparing an ophthalmic composition according to claim 4 wherein said composition comprises a container for dispensing eyedrops and, contained therein, an ophthalmically acceptable solution of a compound as defined in claim 4.

6. A process according to claim 4 for preparing an ophthalmic composition which is an ointment.

7. A process according to any one of claims 4 to 6 wherein the composition contains a compound wherein $R_1$ has 2-4 carbon atoms.

8. A process according to claim 7 wherein $R_1$ is acetyl and $R_2$ is OH, the compound being (5Z,9α,13E,15S)-9,11,15-triacetoxyprosta-5,13-dienoic acid.